# EUROPEAN PATENT APPLICATION

(11) **EP 0 566 897 A2**
(43) Date of publication of application: **27.10.1993**
(21) Application number: 93105016.5
(22) Date of filing: 26.03.1993
(51) Int. Cl.: C12N 9/10, C12N 15/54, C12P 35/00, C12R 1/75

(54) **The complete gene (cefG) encoding the acetyl-CoA: deacetylcephalosporin C acetyltransferase of Cephalosporium acremonium, its isolation and use**

(30) Priority: 07.04.1992 EP 92105986
(71) Applicant: HOECHST AKTIENGESELLSCHAFT, D-65926 Frankfurt am Main (DE)
(72) Inventor: Martin Martin, Juan Francisco, Prof. Dr., E-24004 Leon (ES); Gutierrez Martin, Santiago, E-24005 Leon (ES); Velasco Alvarez, Javier, E-49003 Zamora (ES); Fernandez Perrino, Francisco José, E-47004 Valladolid (ES)

(57) **Abstract**

The gene (cefG) encoding the acetyl-CoA: deacetylcephalosporin C acetyltransferase (DAC-ATF) of Cephalosporium acremonium (syn. Acremonium chrysogenum) C10 has been isolated. It contains two introns and encodes a protein of 444 amino acids with a molecular weight of 49269 that correlates well with the molecular weight deduced by gel filtration (Mol. weight 52 000 ± 1 000). The cefG gene is linked to the cefEF gene (encoding the bifunctional deacetoxycephalosporin C synthase/hydroxylase) but it is expressed in the opposite orientation to the cefEF gene. The isolated cefG complements the deficiency of deacetylcephalosporin acetyltransferase e.g. in the non-producer mutant C. acremonium ATCC 20371 and restores cephalosporin biosynthesis in this strain. The cefG is therefore useful to improve cephalosporin C production.

## Description

The gene (cefG) encoding the acetyl-CoA: deacetylcephalosporin C acetyltransferase (DAC-ATF) of Cephalosporium acremonium (syn. Acremonium chrysogenum) C10 has been isolated. It contains two introns and encodes a protein of 444 amino acids with a molecular weight of 49269 that correlates well with the molecular weight deduced by gel filtration (Mol. weight 52 000 ± 1 000). The cefG gene is linked to the cefEF gene (encoding the bifunctional deacetoxycephalosporin C synthase/hydroxylase) but it is expressed in the opposite orientation to the cefEF gene. The isolated cefG complements the deficiency of deacetylcephalosporin acetyltransferase e.g. in the non-producer mutant C. acremonium ATCC 20371 and restores cephalosporin biosynthesis in this strain. The cefG is therefore useful to improve cephalosporin C production. The last step of the cephalosporin biosynthetic pathway involves the conversion of deacetyl cephalosporin (DAC) to cephalosporin C by the enzyme DAC-ATF (Fig. I). Until recently this enzyme was poorly known and has not been fully purified. The European published patent application EP-A1-0,450,758 claims isolation of the gene encoding DAC-ATF. According to this European application DAC-ATF consists of two subunits with a molecular weight of 27 000 ± 2 000 dalton (subunit) and 14 000 ± 2 000 dalton (subunit 2) as measured by SDS polyacrylamide gel electrophoresis. The probes used for screening of the DAC-ATF cDNA were taken from the N-terminal portion of each subunit 1 and 2 which were determined to be

(subunit 1)    Leu-X-Ala-Gln-Asp-Ile-Ser-Leu-Phe-Thr-Leu-Glu-Ser-Gly-Val-Ile-Leu-
Arg-...
(subunit 2)    Asp-Ser-Gly-Asn-Ser-His-Arg-Ala-Gly-Gln-Pro-Ile-Glu-Ala-Val-Ser-Ser-
Tyr-Leu-Arg-Tyr-Gln-Ala-Gln-Lys-Phe-Ala-...

X stands of a then unidentified amino acid and proved to be Asp. The DAC-ATF encoding gene isolated with these probes was thought to code for a protein molecule having 387 amino acids. A slightly larger molecule starting with Met-Ser-Pro-Gln-Ile-Ala-Asn-Arg-Phe-Glu-Ala-Ser held to be a pre-sequence before the Leu-Asp-Ala-Gly-Asp... start determined for the amino-terminal portion of subunit, was also described. Hence a DNA encoding the protein above or a slightly larger DNA was used to construct plasmids suitable for introducing the allegedly complete DAC-ATF gene into Cephalosporium strains.

Surprisingly it was found that the complete cefG gene encodes a protein of 444 amino acids with a deduced molecular weight of 49269 daltons. The nucleotide sequence of this region shows an open reading frame (ORF) of 1332 basepairs (bp) with a GC content of 56.8 % corresponding to a 1.4 kb transcript. The nucleotide sequence of the complete cefG gene including the intergenic region with the cefEF gene (see examples) and the 5' region of the cefEF gene is shown in Table 1.

The cefG contains two introns. Computer analysis of the cefG nucleotide sequence revealed the presence of two putative introns which showed very good homology to the intron/exon junction sequences and internal consensus sequences involved in the formation of the lariat intermediate in the splicing reaction of the introns. To confirm the presence of the introns three oligonucleotides L, M, N (see below) were used to hybridize with total RNA of C. acremonium. Probes L and M correspond to sequences internal to the putative introns 1 and 2, respectively, whereas probe N corresponds to a translated region (nucleotides 2016 to 2035 in Table 1). Hybridization results showed that probes L and M do not hybrizide with total RNA of C. acremonium whereas probe N gives a clear hybridization signal. These results confirmed the presence of two introns that separate three exons extending from amino acids 1 to 187, 188 to 302 and 303 to 444.

Intron A of the C. acremonium cefG gene corresponds to a region that is missing in the DNA of S. cerevisiae met2 gene. Intron B corresponds to a region with little conservation in the met2 genes of either A. immersus or S. cerevisiae.

The 3'-noncoding region contains a classical AATAAA polyadenylation sequence (nucleotide positions 2845-2852 in Table 1. Analysis of the 5' region upstream from ATG start codon Table 1 reveals a sequence (TACTAT, nucleotide positions 1001 - 1006) related to the consensus "TATA" box 
A T
(TAT A ).
T A
No consensus "CAAT" box (GGPyCAATCT) is observed. These sequences are used for transcription initiation in higher organisms but are not always present in promoters of filamentous fungi. A pyrimidine rich (66 %) stretch (nucleotides 865 - 895) which seems to be characteristic of fungal promoters is present in the upstream region of the cefG gene.

Expression of the cefG gene in C. acremonium was accomplished via complementation of a C. acremonium mutant deficient in DAC-acetyltransferase. The cefG gene in the 7.2 kb BamHI fragment was subcloned in the fungal vector pULJL43 digested with BamHI and dephosphorylated. This construction was used to transform C. acremonium ATCC20371, a cephalosporin-deficient mutant (Table 2) that accumulates DAC (Table 3) and is deficient in the DAC-acetyltransferase. The levels of cephalosporin C and DAC were measured in the untransformed culture and in three transformants, C. acremonium 371.1, 371.2 and 371.3, and a control clone C. acremonium 371P43 (transformed with the control vector pULJL43 without insert).

The results (Tables 2 and 3) show that by introduction of the complete cefG gene production of cephalosporin C takes place.

The present invention concerns therefore the complete cefG gene and its use for the improvement production of cephalosporin C since large amounts of DAC are know to remain unconverted to cephalosporin C in industrial cephalosporin-producing strains.

Northern analysis with probes corresponding to the genes cefEF and cefG indicate that, in 48 h cells, the relative intensity of hybridization is much smaller for the DAC-acetyltransferase than for the expandase/hydroxylase transcript. Since the probes were homologous in both cases and the same RNA preparation was used, these results indicate that the cefG gene (encoding a late enzyme of the cephalosporin pathway) is still poorly expressed at 48 h of incubation. This late expression correlates well with the late conversion of DAC to cephalosporin in cephalosporin fermentations. The same difference in expression of the cefEF and cefG genes was observed in the low-producer strain CW19 and in the high cephalosporin producer C. acremonium C10. Since both, the cefEF and cefG are expressed divergently from the same promoter region, a differential mechanism of control of these late genes of the pathway must occur.

The isolated complete cefG gene thus permits optimization of cefG expression by e.g. transformation with plamids expressing this gene so as to increase conversion of DAC to cephalosporin C.

The instant invention is further described in the examples and claims.

### Examples:

### 1. Isolation of the complete cefG gene

C. acremonium C-10, which is a high producer of cephalosporin C (Ramos, F. R. et al., FEMS Microbiol. Lett. 35, p. 123 - 127 (1986)) was used as the source of DNA. A gene library of C. acremonium C10 was constructed in the ble-EMBL3 vector as described before (Gutiérrez et al., J. Bacteriol. 173, p. 2354 - 2365 (1991)). Screening of phages for the cefEF gene was done with a 30-mer oligonucleotide 5'-GGCAAGTACTCGGACTACTCGACGTGCTAC-3' (probe K) that was synthesized according to the nucleotide sequence of the cefEF gene of C. acremonium (nucleotide positions 274 to 303; Samson et al., Bio/Technology 5, p. 1207 - 1214 (1987)). Three other probes L (5'GGATCGGTGCGCTTACC-3'), M (5'-TGAGCATCGACCGACGGCAA-3') and N (5'-TACTCCCCGTCCAGGTACTT-3') were used to confirm the presence of two different introns in the cefG gene.

The oligonucleotides were labelled at their 5'-ends with polynucleotide kinase as described previously (Diéz et al., J. Biol. Chem. 265, p. 16358 - 16365 (1990)). Southern hybridizations were carried out by standard procedures using a prehybridization buffer containing 6 x SSC, 2 x Denhardt's solution with 0.25 % SDS.

Fragments of the gene subcloned into Bluescript® KS(+) vector were sequenced by generating ordered sets of deletions using the erase-a-base system (Promega, Madison, WI) by digestion with exonuclease III (S. Henitzoff, Gene 28, p. 351 - 359 (1984)). Sequencing of the ordered sets of fragments was carried by the dideoxynucleotide method using either Sequenase® (U. S. Biochemicals, Cleveland, Oh) or Taq polymerase (Promega, Madison, WI) as described previously (Montenegro et al., Mol. Gen. Genet. 221, p. 322 - 330 (1990)).

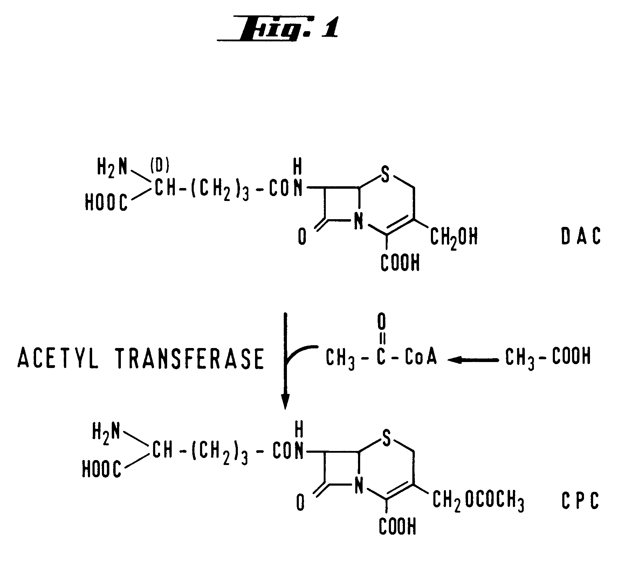

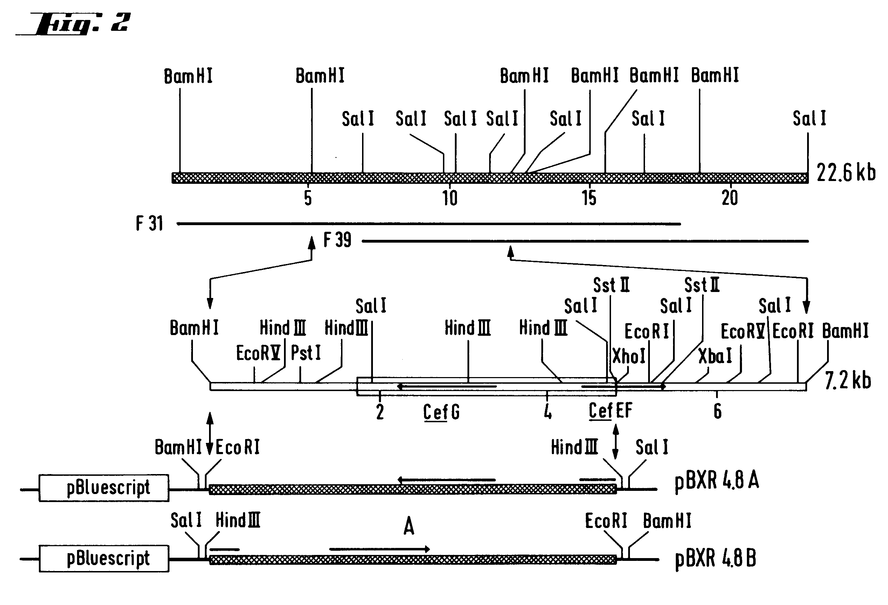

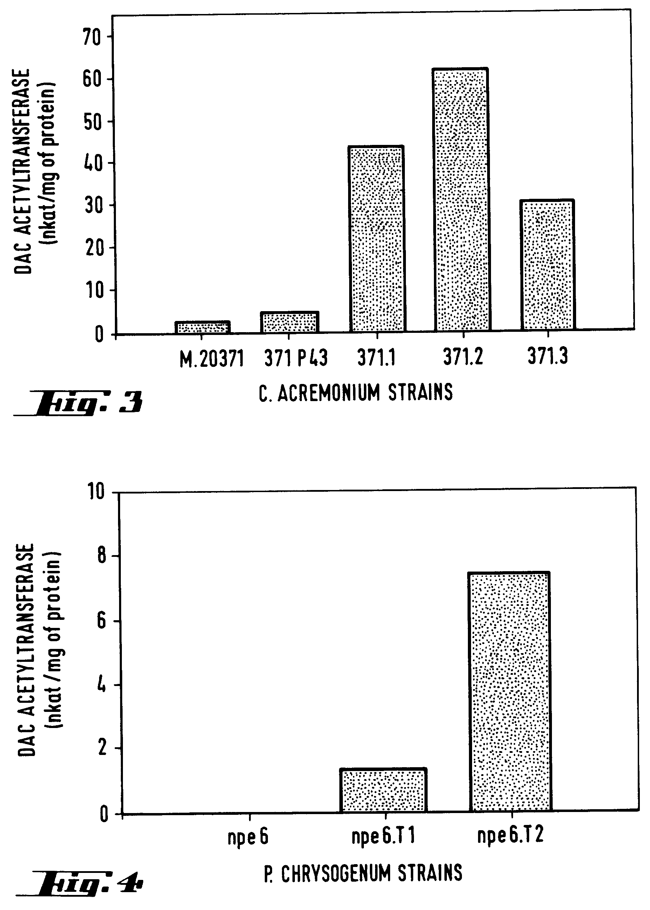

The results are summarized in Fig. 2 and Table 1. The complete cefG gene could be located in a DNA region flanking the cefEF gene. Clones carrying the expandase/hydrolase (cefEF) gene were selected by hybridization with the 30 mer probe K (see above). Two clearly hybridizing phages F31 and F39 were selected and purified. Both phages showed overlapping DNA inserts with a common SalI band of 2.7 kb. A 7.2 kb BamHI fragment of phage F31 that hybridized with probe K was subcloned in Bluescript® and mapped (Fig. 2). A fragment around the SstII site (300 bp) was sequenced confirming that the cloned fragment included the expandase/hydrolase gene. In Fig. 2 the arrows show the position and length of the respective cefEF and cefG genes, which are expressed in opposite orientations. The boxed area in the 7.2 kb BamHI fragment was sequenced. A 4.8 kb fragment A was subcloned with filled ends in both orientations to give pBXR4.8A and pBXR4.8B. Table 1 shows the nucleotide and deduced amino acid sequence of a 3.11 kb DNA region that includes the complete cefG gene and the upstream and downstream intergenic sequences. The ATG translation initiation triplet of the cefEF and cefG genes are boxed. The polyadenylation sequence AAATAAA downstream from the cefG gene and the intron consensus sequences are underlined. A pyrimidine stretch in the promoter region and a TATA box-like sequence are overlined. Note that the cefEF gene is expressed in the opposite orientation from the complementary strand.

### 2. Expression of the complete cefG gene in a C. acremonium mutant deficient in DAC-ATF

The cefG gene in the 7.2 kb BamHI fragment was subcloned in the fungal vector pULJL43 digested with BamHI and dephosphorylated. This construction was used to transform C. acremonium ATCC20371, a cephalosporin-deficient mutant (Table 2) that accumulates DAC (Table 3) and is deficient in the DAC-acetyltransferase (Fig. 3). The levels of cephalosporin C and DAC were measured in the untransformed culture and in three transformants, C. acremonium 371.1, 371.2 and 371.3, and a control clone C. acremonium 371P43 (transformed with the control vector pULJL43 without insert).

Results (Tables 2 and 3) indicate that C. acremonium 20371 does not produce significative amounts of cephalosporin C either untransformed or when transformed with the control plasmid pULJL43, but instead it accumulated considerable amounts of DAC. Transformants 371.2 and 371.3 (complemented with the cefG gene) regained the ability to synthesize cephalosporin C and instead they did not accumulate any detectable levels of DAC. Transformant strain 371.1 formed cephalosporin C but still retained considerable amounts of DAC suggesting that it may have low expression of the acetyltransferase gene (see page 4 and the results of northern blots).

The non-producer mutant ATCC20371 showed a residual DAC-acetyltransferase activity (with or without transformation with the pULJL43 plasmid vector) after 96 h of culture whereas the three transformed clones showed up to 10 to 15-fold higher levels of activity (Fig. 3). A good correlation was observed between the conversion of DAC to cephalosporin C as deduced from comparison of Tables 2 and 3, and the DAC-acetyltransferase activity in the different transformants; transformant 371.3 showed lower levels of acetyltransferase than the other transformants.

Quantification of cephalosporin C and deacetyl-cephalosporin C was performed as follows:
Cultures of the different strains and transformants of C. acremonium were grown for production of cephalosprin C or DAC in defined production medium as described previously (D. H.Zanca and J. F. Martín, J. Antibiot. 36, p. 700 - 708 (1983)). One ml of the culture broths was taken every 24 h and filtered through Millipore 10 000 NMWL to remove molecules of molecular weight above 10 000 dalton. Aliquots (50 µl) of the filtered broth were used for cephalosporin analysis in a Beckman System Gold HPLC equipped with a µBondapack® C18 column (300 x 4 mm). DAC and cephalosporin C were eluted with a mixture of solvents A) 10 mM acetic acid-sodium acetate, pH 4.7 and B) acetonitrile (100 %) using a gradient of solvent B as follows:
time 0 → 5 min → 10 min → 20 min → 25 min
% solvent B 0 % → 0 % → 5 % → 5 % → 0 %
with a constant flow of 1.3 ml/min. Under these conditions DAC eluted with a retention time of 3.4 min and cephalosporin C at 12.8 min. Both compounds were identified by co-elution in the HPLC with authentic samples of cephalosporin C and DAC (provided by F. Salto, Antibióticos, S. A., León, Spain).

The DAC-acetyltransferase activity was assayed in a reaction mixture containing 50 µl acetyl-CoA 5 mM (Sigma Chem. Co., St. Louis, Mo), 50 µl DAC 5 mM [or deacetyl-7-amino cephalosporanic acid (deacetyl-7-ACA)], 25 µl MgSO₄ 50 mM, and cell-free extract (100 g of protein) in 150 µl of 50 mM potassium phosphate buffer ph 7.0. The enzyme is able to convert the substrate analog deacetyl-7-ACA to the acetylated derivative 7-ACA.

The mixture was incubated for 1 h at 37° C and the reaction was stopped by quick cooling in ice and filtering the iced reaction mixture through Millipore 10 000 NMWL filters. Fifty µl of the reaction mixture were injected in HPLC and the cephalosporin C (or 7-ACA) were eluted using the following program:
time 0 → 2 min → 10 min → 15 min → 20 min → 25 min
% solvent B 0 % → 3 % → 3 % → 7 % → 7 % → 0 %
7-ACA eluted with a retention time of 7.1 min whereas the deacetylated substrate deacetyl-7-ACA showed a retention time of 2.5 min..

### 3. Expression of the cefG gene in P. chrysogenum

Heterologous expression of the cefG gene was observed in P. chrysogenum npe6. This is a mutant blocked in penicillin biosynthesis because it lacks isopenicillin N acyltransferase. No DAC-acetyltransferase was observed in this strain whereas transformants npe6.T1 and npe6.T2 showed respectively 1.35 and 7.4 nkatals of DAC-acetyltransferase/mg of protein (Fig. 4). These two transformants failed to synthesize cephalosporin although they carried genes encoding both, the expandase/hydroxylase and the DAC-acetyltransferase. This is an indication that the isopenicillin epimerase gene (missing to complete the cephalosporin pathway) is not located on the 7.2 kb BamHI fragment used to transform P. chrysogenum or at least it is not functional. Furthermore, they did not produce penicillin what indicated that DAC-acetyltransferase can not replace the isopenicillin N acyltransferase to synthesize penicillins.

TABLE 1

l g r r a n r d a l t v a r k e e e s g n k f f d v c t e r a s t
TTCGGCGCCGCCCGCAATGCCAGCCGCTCGCAGTGCCGGGAGAAGAGGAGGAGCGAAGGCAAGAACTTTTTCAGTTGCGTGCAGAGTGCGCGGCTCCACA 100
h d d d v l g s e t l y f i g k t t v a e a l e t l v k g s k l d d
CCAGCAGCAGCTGGTCCGGCGAGAGCCAGTTCATCTTCTATGGGAACCACCACTGCCGGAGCCGCTCGAGCCACTCCTGGAACGGCGAGAACTCCAGCAG 200
l r f v p v k s t m
CTCTGCTTTCTGCCCCTGGAACCTTCAGTA
GTTGATGCTGTGGTTTTGAGCGATGACTTGAGAGGAGTAGCGTGGAGGAAAGTTCTGCAAGAGGAATTTA 300
AGGATTCACAAGATCCCAGTGAGAACGAAACGTTGTCAAAGCGGTATATATATATCTCAAACCCCACCTCGTAGCTTACGCCGAGGAACTCCTTTTTAGA 400
CAACTGCTACTTAGCCGTAAGTGACGCCCTGCTTCCCCTCAGCCTTGGCCGCACACGTCAATGTAGCATTGTAAACCCACGAGTGTCTTGTGAAGTTTTG 500
TCAACGAATCATAAGAAGCCATCGAGTTCTCTTCTCGTTCTTGGTTCGCAGGAGAATATGTATCGTGCATGGTCCCTGATCGTCGAGACCGCCATGGAAT 600
CGTGCAAGCCTTAATTCTCCGTACAAGCTTCCCCATTCGGACAAGATTGCGATGATGTGGATGCGGGCTCTTTTAATAAGGACCTTCTTAACCGATGGTC 700
CGAGAGTGCCTAGGACGGGTCCATGTGCATACACGACGGACCCTCGACCTCCTATTAGGAGCATGAGGGACGACAAAATGCGAACGACGATGCATCAAAA 800
TGCACTGCAACGTCGAGTTGTGGGCTACTCGCCTTCTGATTCGCAAGCCCTCGGCGAGTCCACCTACTAGTAGCTTGGGAATAAACAGCAAGTTTCGCCG 900
CCAAAAGGGCTGCCCGGCATCCGATTCGATGCCATTGTACATCAAGTCGGAAATGGTGCTCCGTTTCCCCCTGGGGTGAGAGGGCGAAGGAGTAGTTCGA 1000
CCAGTCGCAGCCGCACCCAGAGCCGCAGGTTTTATCGGATGTTGCTTCGATCCGATCGTATCCCGCGCGGCCTAGATCTTGCTAATACGAGTCGGAGAGT 1100
TACTATTCCGGGCTTATGCGGACGGGCCGCCGCCGTCGATGCCGGCCAAGGCTTGTCGTGCATGATAGATGCTGCCGTCGGCCCAAGTGGCCCGTCTAAA 1200
m l p s a q v a r l k
GCCGGACCCCTTTCCCCCGAGTCTCTCCCCGATCCCGCACGGGGCCGTCACTTTCGCTGCCCTCGCTCCTTGTCATAACCTACCTATATTCTCATCCCGG 1300
p d p f p p s l s p i p h g a v t f a a l a p c h n l p i f s s r
CAAATGCTGCGGGATAGCCTCACCTACAGCCACACGTCGCCCACCATGTCGCCTCAGATCGCCAATCGCTTCGAGGCTTCGCTAGATGCCCAAGACATAG 1400
q m l r d s l t y s h t s p t m s p q i a n r f e a s l d a q d i a
CCAGAATATCGCTCTTCACACTGGAATCTGGCGTCATCCTTCGCGATGTACCCGTGGCATACAAATCGTGGGGTCGCATGAATGTCTCAAGGGATAACTG 1500
r i s l f t l e s g v i l r d v p v a y k s w g r m n v s r d n c
CGTCATCGTCTGCCACACCTTGACGAGCAGCGCCCATGTCACCTCGTGGTGGCCCACACTGTTTGGCCAAGGCAGGGCTTTCGATACCTCTCGCTACTTC 1600
v i v c h t l t s s a h v t s w w p t l f g q g r a f d t s r y f
ATCATCTGCCTAAATTATCTCGGGAGCCCCTTTGGGAGTGCTGGACCATGTTCACCGGACCCCGATGCAGAAGGCCAGCGCCCGTACGGGGCCAAGTTTC 1700
i i c l n y l g s p f g s a g p c s p d p d a e g q r p y g a k f p
CTCGCACGACGATTCGAGATGATGTTCGGTAGGTAAGCGCACCGATCCAGCTTGTCTCAATATCGAGTGGTCAGGACAATCCAGGCTAAGCTTTCCGTGT 1800
r t t i r d d v r
CCAAAAGTATTCATCGCCAGGTGCTCGACAGGTTAGGCGTCAGGCAAATTGCTGCCGTAGTCGGCGCATCCATGGGTGGAATGCACACTCTGGAATGGGC 1900
i h r q v l d r l g v r q i a a v v g a s m g g m h t l e w a
CTTCTTTGGTCCCGAGTACGTGCGAAAGATTGTGCCCATCGCGACATCATGCCGTCAGAGCGGCTGGTGCGCAGCTTGGTTCGAGACACAGAGGCAGTGC 2000
f f g p e y v r k i v p i a t s c r q s g w c a a w f e t q r q c
ATCTATGATGACCCCAAGTACCTGGACGGGGAGTACGACGTAGACGACCAGCCTGTCCGGGGGCTCGAAACAGCGCGCAAGATTGCGAATCTCACGTACA 2100
i y d d p k y l d g e y d v d d q p v r g l e t a r k i a n l t y k
AGAGCAAACCTGCGATGGACGAGCGCTTCCATATGGCTCCAGGAGTCCAAGCCGGTGAGTTTATAGATGCCTTGCCGTCGGTCGATGCTCAGAGCTAATC 2200
s k p a m d e r f h m a p g v q a
AGACCGAACCCGCTGCTAGGCCGGAATATCAGCAGCCAGGATGCGAAGAAGGAAATCAACGGCACAGACAGCGGCAACAGCCACCGTGCTGGCCAGCCCA 2300
g r n i s s q d a k k e i n g t d s g n s h r a g q p i
TTGAAGCCGTATCTTCCTATCTCCGGTACCAGGCCCAGAAGTTTGCCGCGAGCTTCGACGCCAACTGCTACATCGCCATGACACTCAAGTTCGACACCCA 2400
e a v s s y l r y q a q k f a a s f d a n c y i a m t l k f d t h
CGACATCAGCAGAGGCCGGGCAGGATCAATCCCGGAGGCTCTGGCAATGATTACACAACCAGCGTTGATCATTTGCGCCAGGTCAGACGGTCTGTACTCG 2500
d i s r g r a g s i p e a l a m i t q p a l i i c a r s d g l y s
TTTGACGAGCACGTTGAGATGGGGCGCAGTATCCCAAACAGTCGTCTTTGCGTGGTGGACACGAATGAGGGTCATGACTTCTTTGTAATGGAAGCGGACA 2600
f d e h v e m g r s i p n s r l c v v d t n e g h d f f v m e a d k
AGGTTAATGATGCCGTCAGAGGATTCCTCGATCAGTCATTAATGTGAGGCTATGGAGGTGTCAGCCTGCCGGTGCGCGTACTTGCCAGGGTGATCGATGT 2700
v n d a v r g f l d q s l m .
ACTCTCAGATAGTCTCCATGTGAGTATGGATTTCGCTGTTTCCGCTCGGATATAGGCACTCTCAGGCCATCTCGCAGTAGGTATCAGAACAGCAGCTGAG 2800
GCCTTCTCGTAAAGTAGGTTGTGTCAATAGATTCATAAAGCGTCAAATAAAGCCCAAAGTCGCAGTAGACTCATCGCATCGCAAGTCTCAGAGGGTCGAC 2900
TCGGCAGATTCGAGGCATTGTAGCACATTGTCGAGGCATTGAGGCGGAGACTTGACCCATCCAACTCGGCCAGAGGAGCCAGGCAAAGCATCTCAGCGTA 3000
GGCTCCATGCAAACATGCGTGGCTCAACTCAGCAAGCTCATTGCCAACGAGGTGAAAGAAAATAGAAGGTAGCGGAGGCAGGCGGGTATCGTAGTAACAC 3100
CGTCCAATAA
3110

**Table 2**

| Complementation of the deficiency of cephalosporin C biosynthesis of mutant *C. acremonium* M20371 with the cefG gene in several transformants | | | | | |
|---|---|---|---|---|---|
| Strains | Cephalosporin C (µg/ml) at | | | | |
| | 0 h | 24 h | 48 h | 72 h | 96 h |
| *C. acremonium* M20371 | 0 | 0 | 0 | 0 | 2.7 |
| *C. acremonium* 371.p43 | 0 | 0 | 0 | 9.7 | 15.4 |
| *C. acremonium* 371.1 | 0 | 0 | 11.5 | 4.3 | 111.3 |
| *C. acremonium* 371.2 | 0 | 0 | 17.8 | 47.3 | 124.4 |
| *C. acremonium* 371.3 | 0 | 0 | 66.0 | 13.3 | 64.3 |

**Table 3**

| Accumulation of deacetylcephalosporin C by *C. acremonium* M20371 and different clones transformed with the *cef*G gene | | | | | |
|---|---|---|---|---|---|
| Strains | Deacetylcephalosporin C (µg/ml) at | | | | |
| | 0 h | 24 h | 48 h | 72 h | 96 h |
| *C. acremonium* M20371 | 0 | 0 | 0 | 37.0 | 19.7 |
| *C. acremonium* 371.p43 | 0 | 0 | 29.7 | 101.4 | 28.3 |
| *C. acremonium* 371.1 | 0 | 0 | 72.6 | 88.4 | 53.6 |
| *C. acremonium* 371.2 | 0 | 0 | 3.4 | 0.3 | 0 |
| *C. acremonium* 371.3 | 0 | 0 | 0 | 0 | 0 |

## Claims

1. The complete gene (cefG) encoding the acetyl-CoA:
deacetylcephalosporin C acetyltransferase/DAC-ATF of Cephalosporium acremonium.

2. The cefG gene of claim 1, characterized by the nucleotide sequence of Table 1.

3. cDNA encoding the cefG gene of claim 1 or 2.

4. A Plasmid comprising the cefG gene of claims 1, 2 or 3.

5. A Plasmid according to claim 4, characterized in that the cefG gene is under control of an independent and inducible promotor.

6. Microorganism capable of producing cephalosporin C characterized in that it is transformed with a plasmid according to claim 4 or 5.

7. Cephalosporium acremonium strain, transformed with a plasmid according to claim 4 or 5.

8. A process of producing cephalosporin C comprising fermentation of a microorganism according to claim 6 or 7 and isolating the cephalosporin C so produced.

9. Method of using the complete cefG gene of claim 1 or 2 for improving the production of cephalosporin C.
